(19) Europäisches Patentamt
European Patent Office
Office européen des brevets

(11) **EP 3 789 852 B1**

(12) **FASCICULE DE BREVET EUROPEEN**

(45) Date de publication et mention de la délivrance du brevet:
**23.03.2022  Bulletin 2022/12**

(21) Numéro de dépôt: **20194409.7**

(22) Date de dépôt: **03.09.2020**

(51) Classification Internationale des Brevets (IPC):
**G06F 3/01** *(2006.01)*    **G06F 3/038** *(2013.01)*
**A61B 5/369** *(2021.01)*

(52) Classification Coopérative des Brevets (CPC):
**G06F 3/015; A61B 5/369; G06F 3/038;**
A61B 2560/0233

(54) **MÉTHODE DE CALIBRATION ITÉRATIVE D'UNE INTERFACE NEURONALE DIRECTE UTILISANT UN MÉLANGE MARKOVIEN D'EXPERTS À RÉGRESSION MULTIVARIÉE**

ITERATIVE METHODE ZUR KALIBRIERUNG EINER DIREKTEN NEURONALEN SCHNITTSTELLE UNTER VERWENDUNG EINER EXPERTEN-MARKOV-MISCHUNG MIT MULTIVARIATER REGRESSION

METHOD FOR ITERATIVE CALIBRATION OF A DIRECT NEURAL INTERFACE USING A MARKOV MIXTURE OF EXPERTS WITH MULTIVARIATE REGRESSION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **04.09.2019  FR 1909706**

(43) Date de publication de la demande:
**10.03.2021  Bulletin 2021/10**

(73) Titulaire: **Commissariat à l'énergie atomique et aux énergies alternatives**
**75015 Paris (FR)**

(72) Inventeurs:
• **MOLY, Alexandre**
**38054 GRENOBLE CEDEX 9 (FR)**
• **AKSENOVA, Tetiana**
**38054 GRENOBLE CEDEX 9 (FR)**

(74) Mandataire: **Brevalex**
**56, Boulevard de l'Embouchure**
**B.P. 27519**
**31075 Toulouse Cedex 2 (FR)**

(56) Documents cités:
**FR-A1- 3 046 471    FR-A1- 3 053 495**
**FR-A1- 3 061 318**

• **ANDREY ELISEYEV ET AL: "Recursive Exponentially Weighted N-way Partial Least Squares Regression with Recursive-Validation of Hyper-Parameters in Brain-Computer Interface Applications", NATURE, SCIENTIFIC REPORTS, vol. 7, no. 1, 24 novembre 2017 (2017-11-24), XP055705682, DOI: 10.1038/s41598-017-16579-9**

**Description**

**DOMAINE TECHNIQUE**

La présente invention concerne le domaine des interfaces neuronales directes encore dénommées BCI (*Brain Computer Interface*) ou BMI (*Brain Machine Interface*). Elle trouve notamment à s'appliquer à la commande neuronale directe d'une machine, telle qu'un exosquelette ou un ordinateur.

**ÉTAT DE LA TECHNIQUE ANTÉRIEURE**

Les interfaces neuronales directes utilisent les signaux électro-physiologiques émis par le cortex cérébral pour élaborer un signal de commande. Ces interfaces neuronales ont fait l'objet de nombreuses recherches notamment dans le but de restaurer une fonction motrice à un sujet paraplégique ou tétraplégique à l'aide d'une prothèse ou d'une orthèse motorisée.

Les interfaces neuronales peuvent être de nature invasive ou non invasive. Les interfaces neuronales invasives utilisent des électrodes intracorticales (c'est-à-dire implantées dans le cortex) ou des électrodes corticales (disposées à la surface du cortex) recueillant dans ce dernier cas des signaux électrocorticographiques (ECoG). Les interfaces neuronales non invasives utilisent des électrodes placées sur le cuir chevelu pour recueillir des signaux électroencéphalographiques (EEG). D'autres types de capteurs ont été également envisagés comme des capteurs magnétiques mesurant les champs magnétiques induits par l'activité électrique des neurones du cerveau. On parle alors de signaux magnétoencéphalographiques (MEG).

Les interfaces neuronales directes utilisent avantageusement des signaux de type ECoG, présentant l'avantage d'un bon compromis entre biocompatibilité (matrice d'électrodes implantées à la surface du cortex) et qualité des signaux recueillis.

Les signaux ECoG ainsi mesurés doivent être traités afin d'estimer la trajectoire du mouvement désiré par le sujet et en déduire les signaux de commande de l'ordinateur ou de la machine. Par exemple, lorsqu'il s'agit de commander un exosquelette, l'interface BCI estime la trajectoire du mouvement désiré à partir des signaux électro-physiologiques mesurés et en déduit les signaux de contrôle permettant à l'exosquelette de reproduire la trajectoire en question. De manière similaire, lorsqu'il s'agit de commander un ordinateur, l'interface BCI estime par exemple la trajectoire souhaitée d'un pointeur ou d'un curseur à partir des signaux électro-physiologiques et en déduit les signaux de commande du curseur/pointeur.

L'estimation de trajectoire, et plus précisément celle des paramètres cinématiques (position, vitesse, accélération), est encore dénommée décodage neuronal dans la littérature. Le décodage neuronal permet notamment de commander un mouvement (d'une prothèse ou d'un curseur) à partir de signaux ECoG.

Lorsque les signaux ECoG sont acquis en continu, l'une des principales difficultés du décodage réside dans le caractère asynchrone de la commande, autrement dit dans la discrimination des phases pendant lesquelles le sujet commande effectivement un mouvement (périodes actives) des phases où il n'en commande pas (périodes de repos).

Pour contourner cette difficulté, des interfaces neuronales directes, dites synchrones, ne donnent la possibilité de commander un mouvement que pendant des fenêtres temporelles bien déterminées (par exemple des intervalles de temps se succédant périodiquement), signalées au sujet par un indice extérieur. Le sujet ne peut alors commander le mouvement que pendant ces fenêtres temporelles, ce qui s'avère prohibitif dans la plupart des applications pratiques.

Plus récemment, des interfaces neuronales directes à décodage continu ont été proposées dans la littérature. L'article de M. Velliste et al. intitulé « Motor cortical correlates of arm resting in the context of a reaching task and implications for prosthetic control » publié dans The Journal of Neuroscience, 23 avril 2014, 34(17), pp. 6011-6022, décrit notamment une interface neuronale directe dans laquelle les périodes de repos (*idle* state) sont détectées par LDA (Linear Discriminant Analysis) à partir de la fréquence d'émission des potentiels d'action (*neuron firing rate*). Les paramètres cinématiques sont estimés pendant les périodes actives au moyen d'un modèle à transition d'états, les états étant prédits au moyen d'un filtre dit de Laplace-Gauss. Toutefois, les résultats ont été obtenus pour des matrices de micro-électrodes et n'ont pu être reproduits pour des électrodes corticales classiques. En outre, les commutations entre les périodes de repos et les périodes actives se traduisent par des discontinuités dans le décodage du mouvement et donc des à-coups le long de la trajectoire.

Une autre approche a été proposée dans l'article de L. Srinivasam et al. intitulé « General-purpose filter design for neural prosthetic devices » publié dans J. Neurophysiol.

Vol 98, pp. 2456-2475, 2007. Cet article décrit une interface neuronale directe à décodage continu dans laquelle la séquence des états actifs et des états de repos est générée par un modèle markovien du 1<sup>er</sup> ordre. Un filtre de Kalman commuté ou SKF (Switching Kalman Filter) dont les matrices d'observation et les matrices de transitions dépendent de la variable de commutation cachée (état actif/ état de repos) permet d'estimer les paramètres cinématiques du mouvement. Ce type d'interface neuronale directe a été utilisé pour commander une chaise roulante à partir de signaux EEG sur la base de données de simulation mais n'a pas été testé pour des signaux ECoG. En outre, la détection d'état (état actif/ état de repos) est quelquefois erronée (taux élevé de faux positifs et faux négatifs).

Enfin, les interfaces neuronales directes précitées ont été développées pour décoder le mouvement d'un seul membre d'un sujet. Elles ne sont donc pas adaptées à la commande d'un exosquelette à plusieurs membres, notamment pour un sujet tétraplégique, paraplégique ou hémiplégique, ce qui limite sensiblement leur champ d'application.

Il a été proposé dans la demande publiée sous le numéro FR-A-3053495, de décoder le mouvement de plusieurs membres au moyen d'un mélange markovien d'experts ou MME (*Markov Mixture of Experts*). Ce décodage repose sur un modèle markovien caché ou HMM (*Hidden Markov Model*), un estimateur (ou expert) étant associé à chaque état caché du modèle.

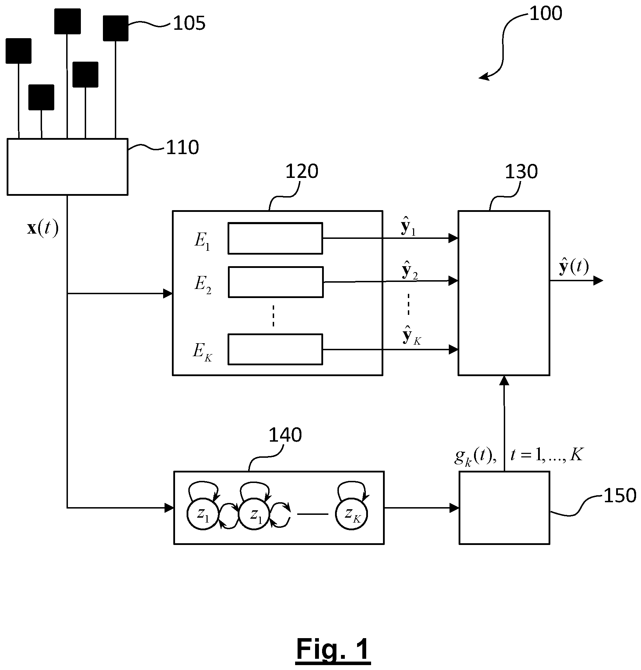

On a représenté schématiquement en Fig. 1, une interface BCI utilisant un décodage continu de mouvement par mélange markovien d'experts.

Cette interface, 100, fait intervenir, d'une part, un automate à états cachés, 140, pouvant prendre $K$ états possibles et, d'autre part, une pluralité $K$ d'estimateurs, 120, chaque estimateur (ou expert) étant associé à un état caché.

Les estimations des différents experts sont combinées dans un module de combinaison (*gating network*), 130, pour donner une estimation, $\hat{\mathbf{y}}(t)$ de la variable à expliquer, $\mathbf{y}(t)$, ici les paramètres cinématiques du mouvement, à partir de l'observation $\mathbf{x}(t)$ représentant les caractéristiques des signaux neuronaux à l'instant $t$, captés au moyen des électrodes 105. La séquence des observations $\mathbf{x}[1:t]=\{\mathbf{x}(1),\mathbf{x}(2),...,\mathbf{x}(t)\}$ est modélisée par un processus markovien sous-jacent, de premier ordre, à émission continue.

Si la variable d'entrée $\mathbf{x}(t)$ est de dimension $M$ et la réponse $\mathbf{y}(t)$ de dimension $N$, et si les modèles prédictifs des différents experts $E_k$, sont choisis linéaires, autrement dit $E_k(\mathbf{x}(t))=\beta_k\mathbf{x}(t)$ où $\beta_k$ est une matrice de taille $N\times M$, l'estimation par mélange d'experts s'exprime comme suit :

$$\hat{\mathbf{y}}(t) = \sum_{k=1}^{K} g_k(t)\boldsymbol{\beta}_k \mathbf{x}(t) \tag{1}$$

où $g_k(t)$ sont les coefficients de pondération (ou de mélange) vérifiant $\sum_{k=1}^{K} g_k(t)=1$ .

Comme indiqué dans la demande précitée, les paramètres du modèle HMM, c'est-à-dire les probabilités de transition entre états cachés, et les matrices $\beta_k$ peuvent être estimés lors d'une phase de calibration, au moyen d'une méthode d'estimation supervisée. Dans ce cas, l'automate HMM et les $K$ experts sont entraînés indépendamment les uns des autres. En particulier, chaque expert $E_k$ peut être entraîné sur une sous-séquence d'observations correspondant à l'état $k$, la matrice $\beta_k$ du modèle prédictif étant alors estimée au moyen d'une régression PLS (*Partial Least Squares*) linéaire à partir de cette sous-séquence.

De manière plus générale, la relation linéaire (1) peut être étendue à une relation multilinéaire. La variable d'entrée peut être alors représentée sous forme tensorielle $\underline{\mathbf{X}} \in \mathbb{R}^{I_1 \times ... \times I_n}$ où $n$ est l'ordre du tenseur d'entrée et $I_i$ est la dimension du mode $i$.

Les modes du tenseur d'entrée peuvent être par exemple le temps (nombre d'échantillons dans le temps), la fréquence (nombre de bandes spectrales d'analyse), l'espace (nombre d'électrodes). De même, la réponse peut être représentée sous forme tensorielle $\underline{\mathbf{Y}} \in \mathbb{R}^{J_1 \times ... \times J_m}$ où $m$ est l'ordre du tenseur de sortie. Les modes du tenseur de sortie peuvent correspondre à différents effecteurs, agissant par exemple sur différentes articulations d'un exosquelette.

Les tenseurs des modèles prédictifs des différents experts peuvent alors être estimés par une méthode PLS multivariée ou NPLS (*N-way PLS*) comme décrit dans la demande publiée sous le numéro FR-A-3046471.

Quels que soient les modèles prédictifs (PLS ou NPLS) des différents experts, on a pu constater qu'en raison de l'absence de stationnarité des signaux dans le cerveau humain, la durée de validité de ces modèles était relativement limitée. Il est par conséquent nécessaire de les mettre à jour régulièrement en procédant à de nouvelles phases de calibration. Or, chaque nouvelle phase de calibration nécessite de traiter une quantité de données très importante, combinant les données des calibrations antérieures et celles de la nouvelle calibration, et ce pour les différents experts. La durée de la mise à jour est alors souvent telle qu'elle requiert une interruption du fonctionnement de l'interface neuronale directe (calibration dite *off-line*). Il a été ainsi proposé dans la demande FR-A-3061318 une méthode itérative de calibration d'une interface neuronale directe basée sur un modèle prédictif NPLS. Cette méthode itérative de calibration a également été décrite dans l'article de A. Eliseyev et al. intitulé « Recursive exponentially weighted N-way Partial Least Squares regression with recursive validation of hyper-parameters in Brain Computer Interface applications » publié dans Nature, Scientific Reports, volume 7, article number 16281, publié le 24.11.2017.

Toutefois cette méthode de calibration itérative ne s'applique pas à une interface neuronale directe utilisant un mélange markovien d'experts comme décrit dans la demande FR-A-3046471.

Un but de la présente invention est par conséquent de proposer une nouvelle méthode de calibration d'une interface neuronale directe utilisant un mélange markovien d'experts tout en ne requérant pas de traiter une quantité considérable de données à chaque phase de calibration, permettant ainsi un fonctionnement de l'interface neuronale directe en temps réel, sans interruption.

## EXPOSÉ DE L'INVENTION

La présente invention est définie par une méthode de calibration d'une interface neuronale directe selon le libellé de la revendication 1 du jeu de revendications ci-après. Des modes de réalisation avantageux sont précisés dans les revendications dépendantes.

## BRÈVE DESCRIPTION DES DESSINS

D'autres caractéristiques et avantages de l'invention apparaîtront à la lecture d'un mode de réalisation préférentiel de l'invention, décrit en référence aux figures jointes parmi lesquelles :

La Fig. 1, déjà décrite, représente de manière schématique une interface neuronale directe utilisant un mélange markovien d'experts, connue de l'état de la technique ;

La Fig. 2 représente sous forme schématique une interface neuronale directe utilisant un mélange markovien d'experts selon un mode de réalisation de l'invention ;

La Fig. 3 représente sous forme d'ordinogramme une méthode de calibration itérative d'une interface neuronale directe à mélange markovien d'experts, selon un mode de réalisation de l'invention.

## EXPOSÉ DÉTAILLÉ DE MODES DE RÉALISATION PARTICULIERS

On considérera dans la suite une interface neuronale directe à décodage continu utilisant un mélange d'experts comme décrit dans la partie introductive.

Les signaux électrophysiologiques issus des différentes électrodes sont échantillonnés et rassemblés par blocs de données, chaque bloc correspondant à une fenêtre glissante d'observation de largeur $\Delta T$. Par exemple, les signaux électrophysiologiques sont échantillonnés à la fréquence de 590 KHz environ, et la taille des blocs de données est de 590 échantillons ($\Delta T = 1$ s), et le décalage $\delta T$ d'une fenêtre d'observation à la suivante est de 59 échantillons ($\delta T = 100$ ms). Chaque fenêtre d'observation est définie par un instant d'observation (*epoch*) auquel démarre la fenêtre en question.

Les signaux électrophysiologiques font ensuite l'objet d'un prétraitement. Ce prétraitement peut notamment comporter une suppression de la moyenne prise sur l'ensemble des électrodes, puis une analyse temps-fréquence est effectuée sur chacune des fenêtres d'observation.

L'analyse temps-fréquence peut utiliser une décomposition en ondelettes, notamment en ondelettes de Morlet.

Ces résultats de l'analyse temps-fréquence peuvent en outre faire l'objet d'un lissage fréquentiel ou d'une décimation.

Ainsi, à chaque fenêtre d'observation, ou instant d'observation $t$, est associé un tenseur d'ordre 3 de données d'observation, d'où la génération d'un tenseur d'entrée d'ordre 4: le premier mode correspond aux fenêtres d'observation successives, le second mode correspond à l'espace, autrement dit aux capteurs, le troisième mode correspond au temps au sein d'une fenêtre d'observation, autrement dit aux positions des ondelettes, et le quatrième mode correspond à la fréquence, autrement dit au nombre de bandes de fréquence utilisées pour la décomposition en ondelettes sur une fenêtre d'observation.

De manière plus générale, le tenseur d'entrée (ou tenseur d'observation) sera d'ordre $n+1$, le premier mode étant dans tous les cas celui relatif aux instants d'observation (*epochs*). Le tenseur d'entrée (ou tenseur d'observation) est noté **X** et est de dimension $N \times I_1 \times ... \times I_n$.

De la même façon, la trajectoire du mouvement imaginé, observé ou réalisé est décrite par un tenseur de sortie (ou tenseur de commande), d'ordre $m+1$, noté $\underline{\mathbf{Y}}$, de dimension $N \times J_1 \times ... \times J_m$, dont le premier mode correspond aux instants successifs auxquels s'appliqueront les commandes (en règle générale, ce premier mode correspond également aux fenêtres d'observation), les autres modes correspondant aux commandes de différents effecteurs ou aux différents degrés de liberté d'un robot multi-axe.

Plus précisément, le tenseur de sortie fournit $N$ blocs consécutifs de données de commande, chacun des blocs permettant de générer les signaux de commande relatifs aux différents effecteurs ou degrés de liberté. Ainsi, on comprendra que la dimension de chaque bloc de données pourra dépendre du cas d'usage envisagé et notamment du nombre de degrés de liberté de l'effecteur.

On notera dans la suite $\underline{\mathbf{X}}_t$ le tenseur d'observation à l'instant $t$. Ce tenseur est par conséquent d'ordre $n$ et de dimension $I_1 \times ... \times I_n$. Il prend ses valeurs dans un espace $\underline{X} \subset \mathbb{R}^{I_1 \times ... \times I_n}$ où $\mathbb{R}$ est l'ensemble de réels. De manière similaire, on notera $\underline{\mathbf{Y}}_t$ le tenseur de commande à l'instant $t$. Ce tenseur de sortie est par conséquent d'ordre $m$ et de dimension $J_1 \times ... \times J_m$. Il prend ses valeurs dans un espace $\underline{Y} \subset \mathbb{R}^{J_1 \times ... \times J_m}$.

On supposera que l'espace $\underline{X}$ est formé par l'union d'une pluralité $K$ de régions, non nécessairement disjointes.

Autrement dit, $\underline{X} = \bigcup_{k=1}^{K} X_k$ où $X_k$, $k = 1,...,K$ sont les régions en question.

L'interface neuronale directe est basée sur un mélange d'experts (ME), chaque expert $E_k$ opérant sur la région élémentaire $X_k$ de l'espace des caractéristiques d'entrée et étant capable de prédire le tenseur de commande $\underline{\mathbf{Y}}_t$ à l'instant $t$ à partir du tenseur d'observation, $\underline{\mathbf{X}}_t$, lorsque ce dernier appartient à $X_k$. Autrement dit, à chaque région $\underline{X}_k$ correspond un modèle de prédiction du tenseur de commande $\underline{\mathbf{Y}}_t$ à partir du tenseur d'observation $\underline{\mathbf{X}}_t$. Un expert $E_k$ peut être ainsi considéré comme une application multilinéaire de $X_k$ dans $\underline{Y}$.

On suppose par ailleurs que chaque expert $E_k$ est associé à un état caché $k$ d'un modèle Markovien du premier ordre (HMM). Les différents experts sont combinés à l'aide de coefficients de combinaison dépendant de l'état caché au moment de la prédiction. En définitive, à partir d'un tenseur d'entrée (ou d'observation) $\underline{\mathbf{X}}_t$, l'interface neuronale estime le tenseur de sortie (ou de commande) $\underline{\mathbf{Y}}_t$, au moyen de :

$$\widehat{\underline{\mathbf{Y}}}_t = \sum_{k=1}^{K} \gamma_k^t \left( \underline{\beta}_k \underline{\mathbf{X}}_t + \underline{\delta}_k \right) \tag{2}$$

où $\underline{\beta}_k$, $\underline{\delta}_k$ sont respectivement un tenseur de coefficients de prédiction et un tenseur de biais de prédiction de l'expert $E_k$ et $\gamma_k^t$ est le coefficient de pondération (encore dénommé *gating coefficient*) relatif à cet expert et à l'instant $t$ considéré.

Le coefficient de pondération $\gamma_k^t$ n'est autre que la probabilité conditionnelle que le modèle HMM soit dans l'état $k$ connaissant les tenseurs d'entrée passés $\underline{\mathbf{X}}_{1:t} = \underline{\mathbf{X}}_1, \underline{\mathbf{X}}_2, ..., \underline{\mathbf{X}}_t$.

L'ensemble des coefficients et des biais de prédiction, dénommés collectivement paramètres de prédiction des différents experts est désigné par $\theta_e = \{(\underline{\beta}_k, \underline{\delta}_k)|k = 1,...,K\}$. L'ensemble de paramètres relatifs à la combinaison des différents experts (incluant les paramètres du modèle HMM sous-jacent) est désigné quant à lui par $\theta_g = \{\mathbf{A}, \{d_k|k = 1,..,K\}, \pi\}$ où $\mathbf{A}$ est la matrice de transition entre états, de taille $K \times K$, c'est-à-dire la matrice dont les éléments $a^{ij}$ (indépendants du temps par hypothèse du modèle HMM) sont définis par $a^{ij} = p(z_t = j|z_{t-1} = i)$ où $z_t$ représente l'état du modèle à l'instant $t$ et $z_{t-1}$ représente l'état du modèle à l'instant précédent ; $\{d_k|k = 1,..,K\}$ les paramètres permettant de déterminer la probabilité conditionnelle d'observer le tenseur d'entrée $\underline{\mathbf{X}}_t$ connaissant l'état $z_t = k$, et $\pi$ est un vecteur de taille $K$ donnant les probabilités d'occupation des différents états à l'instant initial, autrement dit $\pi_i = p(z_t = i)_{t=0}$.

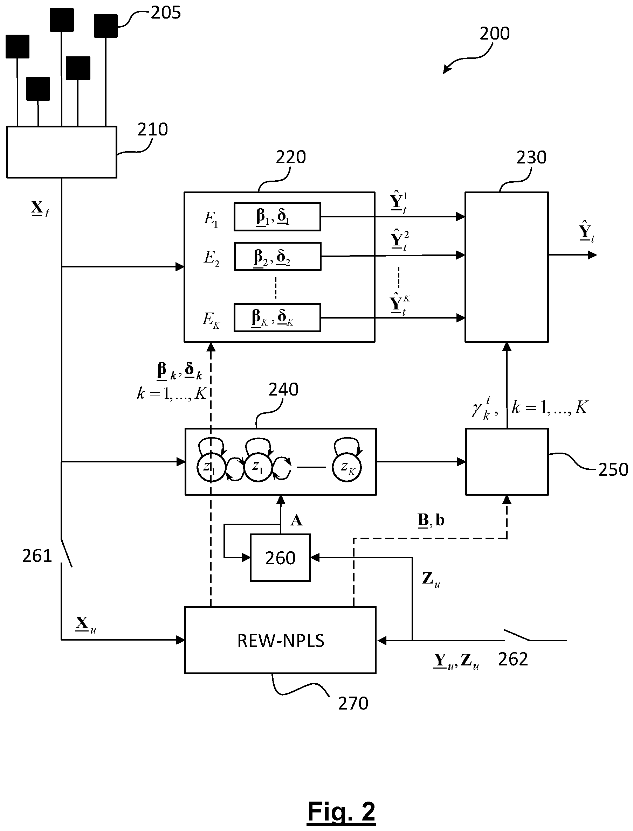

La Fig. 2 représente sous forme schématique une interface neuronale directe utilisant un mélange markovien d'experts selon un mode de réalisation de l'invention.

Les signaux électrophysiologiques issus des différentes électrodes 205 subissent un traitement dans le module de traitement 210. Ce module de traitement effectue un échantillonnage, une suppression optionnelle de la moyenne prise sur l'ensemble des électrodes, puis une analyse temps-fréquence sur chacune des fenêtres d'observation. L'analyse temps-fréquence peut être suivie, le cas échéant, par un lissage fréquentiel ou une décimation, comme indiqué plus haut. Le module de traitement 210 fournit par conséquent un tenseur d'entrée $\underline{\mathbf{X}}_t$ à chaque fenêtre d'observation, caractérisée par un instant d'observation $t$.

L'interface neuronale directe fait intervenir en outre un automate à états cachés, basé sur un modèle HMM, 240, pouvant prendre $K$ états possibles, ainsi qu'une pluralité d'experts, 220, chaque expert étant associé à un état caché.

Chaque expert $E_k$ effectue une estimation $\underline{\mathbf{Y}}_t^k$ du tenseur de sortie $\underline{\mathbf{Y}}_t$ et la fournit à un module de combinaison, 230, qui effectue le mélange des estimations selon l'expression (2). Les coefficients de combinaison (*gating coefficients*) sont déterminés dans le module d'estimation , 250, par $\gamma_k^t = p\left(z_t = k \middle| \underline{\mathbf{X}}_{1:t}\right)$, $k = 1,..,K$, où $z_t$ représente l'état du modèle à l'instant $t$.

L'estimation $\widehat{\underline{\mathbf{Y}}}_t$ donne les différents paramètres cinématiques du mouvement, comme la position, et/ou la vitesse et/ou l'accélération le long de la trajectoire souhaitée.

La calibration de l'interface neuronale est réalisée tout d'abord lors d'une phase d'initialisation puis, lors de phases de calibration ultérieures, à intervalles de temps donnés (calibration en ligne dite aussi *on-line*).

Ces phases de calibration ultérieures sont nécessaires en raison du manque de stationnarité des signaux générés dans le cerveau humain. Elles peuvent être prévues à intervalles réguliers le long de la trajectoire ou des trajectoires successives à exécuter.

Une phase de calibration u fait intervenir une pluralité de blocs de données d'entrée correspondant à une pluralité $\Delta L$ d'instants successifs issus de la séquence d'instants d'observation. On notera $\underline{\mathbf{X}}_u$ le tenseur de dimension $\Delta L \times I_1 \times ... \times I_n$ regroupant cette pluralité de blocs d'entrée. Elle fait intervenir aussi une pluralité de blocs de sortie donnant les paramètres cinématiques de consigne, pris en ces mêmes instants. On notera $\underline{\mathbf{Y}}_u$ le tenseur de consigne de dimension $\Delta L \times J_1 \times ... \times J_m$ regroupant cette pluralité de blocs de sortie.

Les commutateurs 261, 262 sont en position fermée lors d'une phase de calibration. Le module de calibration 270 reçoit le tenseur d'entrée $\underline{\mathbf{X}}_u$, le tenseur de consigne $\underline{\mathbf{Y}}_u$, ainsi que la matrice $\mathbf{Z}_u$ de taille $K \times \Delta L$ dont chacune des colonnes représente l'état de l'automate à l'instant considéré. Plus précisément, si à l'instant $\ell$ l'automate occupe l'état $z_\ell = k$, tous les éléments de la $\ell$ ème colonne de la matrice $\mathbf{Z}_u$ sont nuls excepté celui de la ligne $k$ qui vaut 1.

Le module de calibration calcule à partir de $\underline{\mathbf{X}}_u$, $\underline{\mathbf{Y}}_u$, $\mathbf{Z}_u$ les ensembles de paramètres mis à jour, $\theta_e$ et $\theta_g$.

Au terme de cette phase de calibration, les commutateurs 261, 262 sont ouverts et les ensembles de paramètres $\theta_e$ et $\theta_g$ mis à jour sont respectivement fournis à la pluralité d'experts, 220, et à l'automate 240.

De manière originale, le modèle prédictif de chaque expert $E_k$ est entrainé au moyen d'une régression REW-NPLS (*Recursive Exponentially Weigthed N-way Partial Least Squares*) avec un facteur d'oubli $\lambda^k$ ($0 < \lambda^k < 1$) sur des blocs de données de calibration, $\underline{\mathbf{X}}_u$, $\underline{\mathbf{Y}}_u$, correspondant à l'état caché $z_u = k$, notés $\underline{\mathbf{X}}_u^k$ et $\underline{\mathbf{Y}}_u^k$. Plus précisément, on détermine pour chaque expert $E_k$, et à chaque phase de calibration $u$, les blocs de données $\underline{\mathbf{X}}_u^k$ et $\underline{\mathbf{Y}}_u^k$ permettant de l'entraîner de manière supervisée.

Pour ce faire, on procède comme décrit dans la demande FR-A-3061318, à savoir:

Dans une première étape, on centre et l'on normalise les tenseurs $\underline{\mathbf{X}}_u^k$ et $\underline{\mathbf{Y}}_u^k$ à partir d'un nombre d'observations dit effectif $N_u^k = \lambda^k N_{u-1}^k + N_u^k$ où $N_u^k$ est le nombre de blocs de données correspondant à l'état caché $z_u = k$ lors de la phase de calibration u . On calcule la somme pondérée $s\left(\underline{\mathbf{X}}_u^k\right)$ et la somme quadratique pondérée $sq\left(\underline{\mathbf{X}}_u^k\right)$ :

$$s\left(\underline{\mathbf{X}}_u^k\right) = \lambda^k \sum_{\ell=1}^{N_{u-1}^k} x_{u-1;\ell,i_1,..i_n}^k + \sum_{\ell=1}^{N_u^k} x_{u;\ell,i_1,..i_n}^k \tag{3-1}$$

$$sq\left(\underline{\mathbf{X}}_u^k\right) = \lambda^k \sum_{\ell=1}^{N_{u-1}} \left(x_{u-1;\ell,i_1,..i_n}^k\right)^2 + \sum_{\ell=1}^{N_u^k} \left(x_{u;\ell,i_1,..i_n}^k\right)^2 \tag{3-2}$$

où $x_{u-1;\ell,i_1,..i_n}^k$ et $x_{u;\ell,i_1,..i_n}^k$ représentent respectivement les éléments des tenseurs $\underline{\mathbf{X}}_{u-1}^k$ et $\underline{\mathbf{X}}_u^k$. On en déduit ensuite

la valeur moyenne $\mu\left(\underline{\mathbf{X}}_u^k\right) = \dfrac{s\left(\underline{\mathbf{X}}_u^k\right)}{N_u^k}$ et l'écart-type $\sigma\left(\underline{\mathbf{X}}_u^k\right) = \sqrt{\dfrac{sq\left(\underline{\mathbf{X}}_u^k\right) - \left(\mu\left(\underline{\mathbf{X}}_u^k\right)\right)^2}{N_u^k}}$ . Le tenseur d'entrée centré et normalisé, $\underline{\widetilde{\mathbf{X}}}_u^k$ , a pour éléments :

$$\tilde{x}_{u;\ell,i_1,..i_n}^k = \frac{x_{u;\ell,i_1,..i_n}^k - \mu\left(\underline{\mathbf{X}}_u^k\right)}{\sigma\left(\underline{\mathbf{X}}_u^k\right)} \tag{4}$$

De manière similaire, on calcule la somme pondérée $s\left(\underline{\mathbf{Y}}_{u}^{k}\right)$ et la somme quadratique pondérée $sq\left(\underline{\mathbf{Y}}_{u}^{k}\right)$ :

$$s\left(\underline{\mathbf{Y}}_{u}^{k}\right) = \lambda^{k} \sum_{\ell=1}^{N_{u-1}^{k}} y_{u-1;\ell,j_{1},..j_{m}}^{k} + \sum_{\ell=1}^{N_{u}^{k}} y_{u;\ell,j_{1},..j_{m}}^{k} \qquad (5\text{-}1)$$

$$sq\left(\underline{\mathbf{Y}}_{u}^{k}\right) = \lambda^{k} \sum_{\ell=1}^{N_{u-1}^{k}} \left(y_{u-1;\ell,j_{1},..j_{m}}^{k}\right)^{2} + \sum_{\ell=1}^{N_{u}^{k}} \left(y_{u;\ell,j_{1},..j_{m}}^{k}\right)^{2} \qquad (5\text{-}2)$$

On en déduit ensuite la valeur moyenne $\mu\left(\underline{\mathbf{Y}}_{u}^{k}\right) = \dfrac{s\left(\underline{\mathbf{Y}}_{u}^{k}\right)}{N_{u}^{k}}$ et l'écart-type $\sigma\left(\underline{\mathbf{Y}}_{u}^{k}\right) = \sqrt{\dfrac{sq\left(\underline{\mathbf{Y}}_{u}^{k}\right) - \left(\mu\left(\underline{\mathbf{Y}}_{u}^{k}\right)\right)^{2}}{N_{u}^{k}}}$ ,

puis les éléments du tenseur de sortie centré et normalisé, $\widetilde{\underline{\mathbf{Y}}}_{u}^{k}$ :

$$\tilde{y}_{u;\ell,j_{1},..j_{m}}^{k} = \frac{y_{u;\ell,j_{1},..j_{m}}^{k} - \mu\left(\underline{\mathbf{Y}}_{u}^{k}\right)}{\sigma\left(\underline{\mathbf{Y}}_{u}^{k}\right)} \qquad (6)$$

On définit le tenseur de covariance et le tenseur de covariance croisée, lors de la phase de calibration $u$ , à partir des tenseurs centrés et normalisés $\widetilde{\underline{\mathbf{X}}}_{u}^{k}$ et $\widetilde{\underline{\mathbf{Y}}}_{u}^{k}$ :

$$\mathrm{cov}\left(\widetilde{\underline{\mathbf{X}}}_{u}^{k}, \widetilde{\underline{\mathbf{X}}}_{u}^{k}\right) = \widetilde{\underline{\mathbf{X}}}_{u}^{k} \times_{1} \widetilde{\underline{\mathbf{X}}}_{u}^{k} \qquad (7\text{-}1)$$

$$\mathrm{cov}\left(\widetilde{\underline{\mathbf{X}}}_{u}^{k}, \widetilde{\underline{\mathbf{Y}}}_{u}^{k}\right) = \widetilde{\underline{\mathbf{X}}}_{u}^{k} \times_{1} \widetilde{\underline{\mathbf{Y}}}_{u}^{k} \qquad (7\text{-}2)$$

où $\times_{1}$ désigne le produit tensoriel selon le premier mode (mode temporel d'indice $\ell$) comme décrit dans la demande FR-A-3061318 précitée.

Ces tenseurs de covariance sont modifiés en prenant en compte les tenseurs de covariance de la phase de calibration précédente en les pondérant par le facteur d'oubli $\lambda^{k}$ :

$$\mathrm{cov}\left(\widetilde{\underline{\mathbf{X}}}_{u}^{k}, \widetilde{\underline{\mathbf{X}}}_{u}^{k}\right) = \lambda^{k}\, \mathrm{cov}\left(\widetilde{\underline{\mathbf{X}}}_{u-1}^{k}, \widetilde{\underline{\mathbf{X}}}_{u-1}^{k}\right) + \widetilde{\underline{\mathbf{X}}}_{u}^{k} \times_{1} \widetilde{\underline{\mathbf{X}}}_{u}^{k} \qquad (8\text{-}1)$$

$$\mathrm{cov}\left(\widetilde{\underline{\mathbf{X}}}_{u}^{k}, \widetilde{\underline{\mathbf{Y}}}_{u}^{k}\right) = \lambda^{k}\, \mathrm{cov}\left(\widetilde{\underline{\mathbf{X}}}_{u-1}^{k}, \widetilde{\underline{\mathbf{Y}}}_{u-1}^{k}\right) + \widetilde{\underline{\mathbf{X}}}_{u}^{k} \times_{1} \widetilde{\underline{\mathbf{Y}}}_{u}^{k} \qquad (8\text{-}2)$$

Ce calcul permet de prendre en compte les données d'une phase précédente de calibration, avec un facteur d'oubli, pour mettre à jour les tenseurs de covariance. Les facteurs d'oubli relatifs aux différents experts pourront être choisis identiques $\lambda^{k} = \lambda$, $k = 1,...,K$, où $\lambda$ est alors le facteur d'oubli commun. Alternativement, ils pourront être choisis distincts de manière à offrir plus de flexibilité de prédiction aux différents experts.

A partir des tenseurs de covariance $\mathrm{cov}\left(\widetilde{\underline{\mathbf{X}}}_{u}^{k}, \widetilde{\underline{\mathbf{X}}}_{u}^{k}\right)$ et $\mathrm{cov}\left(\widetilde{\underline{\mathbf{X}}}_{u}^{k}, \widetilde{\underline{\mathbf{Y}}}_{u}^{k}\right)$ on obtient de manière itérative sur le rang $f^k$ = 1,...,$F$ de l'espace des variables latentes (où $F$ est un nombre prédéterminé maximal de dimensions de l'espace des variables latentes), un ensemble de vecteurs de projection $\left\{\mathbf{w}_{u,1}^{k,f^k},...,\mathbf{w}_{u,n}^{k,f^k}\right\}_{f^k=1}^F$, dedimensions respectives $l_1,..,l_n$ un ensemble de tenseurs de coefficients de prédiction, $\left\{\underline{\boldsymbol{\beta}}_u^{k,f^k}\right\}_{f^k=1}^F$, et un tenseur de biais de prédiction, $\left\{\underline{\boldsymbol{\delta}}_u^{k,f^k}\right\}_{f^k=1}^F$, selon la méthode REW-NPLS décrite dans la demande FR-A-3061318 précitée.

Le rang optimal, $f_{opt}^k$, pour chaque expert $E_k$, lors d'une phase de calibration $u$ est déterminé en calculant les erreurs de prédiction de $\underline{\mathbf{Y}}_u^k$ à partir de $\underline{\mathbf{X}}_u^k$ en utilisant les tenseurs de coefficients de prédiction, $\left\{\underline{\boldsymbol{\beta}}_{u-1}^{k,f^k}\right\}_{f^k=1}^F$, et les tenseurs de biais de prédiction, $\left\{\underline{\boldsymbol{\delta}}_{u-1}^{k,f^k}\right\}_{f^k=1}^F$ obtenus lors de la phase de calibration précédente :

$$err_u^{k,f^k} = \lambda^k err_{u-1}^{k,f^k} + \left\|\underline{\mathbf{Y}}_u^k - \hat{\underline{\mathbf{Y}}}_{u-1}^{k,f^k}\right\| \qquad (9)$$

où $\hat{\underline{\mathbf{Y}}}_{u-1}^{k,f^k}$ est l'estimation de $\underline{\mathbf{Y}}_u^k$ obtenue à partir des coefficients de prédiction $\underline{\boldsymbol{\beta}}_{u-1}^{k,f^k}$ et du biais de prédiction $\underline{\boldsymbol{\delta}}_{u-1}^{k,f^k}$ de la phase de calibration précédente.

Le rang $f_{opt}^k$ conduisant à l'erreur de prédiction minimale est alors sélectionné :

$$f_{opt}^k = \arg\min_{f^k=1,..,F}\left(err_u^{k,f^k}\right) \qquad (10)$$

et l'on retient, pour la phase de calibration, le tenseur des coefficients de prédiction et le tenseur de biais de prédiction correspondant à ce rang optimal, autrement dit, à l'issue de la phase de calibration $u$ , on effectue la mise à jour :

$$\underline{\boldsymbol{\beta}}_k = \underline{\boldsymbol{\beta}}_u^{k,f_{opt}^k} \qquad (11\text{-}1)$$

$$\underline{\boldsymbol{\delta}}_k = \underline{\boldsymbol{\delta}}_u^{k,f_{opt}^k} \qquad (11\text{-}2)$$

pour chacun des experts $E_k$, $k$ = 1,..,$K$, de façon indépendante. On obtient ainsi une estimation de l'ensemble de paramètres de prédiction $\theta_e$ au moyen de la méthode de régression REW-NPLS appliquée à chacun des experts.

De manière originale, on estime également les coefficients de combinaison $\gamma_k^t$ , $k$ = 1,..,$K$ au moyen d'une méthode de régression REW-NPLS adaptée au décodage discret, comme décrit plus loin.

Lors d'une phase de calibration, les éléments $a^{ij}$ de la matrice de transition A sont tout d'abord mis à jour de la manière suivante :

$$a_u^{ij} = \lambda a_{u-1}^{ij} + \frac{v_u^{ij}}{K} \qquad (12)$$

où $a_u^{ij}$ (resp. $a_{u-1}^{ij}$ ) sont les éléments de la matrice de transition, estimée pendant la phase de calibration $u$ (resp. $u$-1) et $v_u^{ij}$ est le nombre de transitions de l'état $i$ vers l'état $j$ pendant la phase de calibration $u$ . Ce nombre de transitions est obtenu à partir de la matrice $\mathbf{Z}_u$. Les lignes de la matrice de transition sont ensuite normalisées de manière à ce que la somme des probabilités de transition relatives à un état de départ soit égale à 1.

La matrice de transition $\mathbf{A}$ ainsi mise à jour est fournie par le module de calcul de matrice de transition, 260, au module d'estimation d'état, 240, du modèle HMM.

On définit par $\mathbf{z}_t$ un vecteur de taille $K$ dont les éléments représentent si l'automate est dans l'état $k = 1,..,K$ à l'instant $t$. Ainsi, si l'automate se trouve dans l'état $k$ à l'instant $t$, seul le $k$-ème élément de $\mathbf{z}_t$ sera égal à 1 et les autres éléments seront nuls. On peut considérer $\mathbf{z}_t$ comme un processus à valeurs discrètes dont l'évolution dans le temps suit un modèle prédictif multilinéaire dont la variable d'entrée est le tenseur d'observation $\underline{\mathbf{X}}_t$. Ce modèle prédictif est entraîné de manière supervisée lors de chaque phase de calibration $u$, selon le même principe que le modèle prédictif de chaque expert. Autrement dit :

$$\hat{\mathbf{z}}_t = \underline{\mathbf{B}}\underline{\mathbf{X}}_t + \mathbf{b} \tag{13}$$

où $\hat{\mathbf{z}}_t$ exprime les probabilités $\hat{z}_{k,f}$ que l'automate soit dans l'état $k$ à l'instant $t$, $\underline{\mathbf{B}}$ est un tenseur de coefficients de prédiction de taille $K \times I_1 \times ... \times I_n$ et $\mathbf{b}$ est un vecteur de biais de taille $K$.

Le tenseur $\underline{\mathbf{B}}$ et le vecteur $\mathbf{b}$ sont mis à jour à chaque phase de calibration $u$ au moyen d'une régression REW-NPLS avec un facteur d'oubli $\lambda$, $(0 < \lambda < 1)$ sur la base des observations $\underline{\mathbf{X}}_u$ et $\mathbf{Z}_u$ (matrice de taille $K \times \Delta L$). Ce facteur d'oubli peut être identique au facteur d'oubli commun, lorsqu'un tel facteur d'oubli commun est utilisé pour l'estimation des paramètres de prédiction des différents experts.

On procède de manière itérative sur le rang $f = 1,...,F$ de l'espace des variables latentes à partir du tenseur de covariance $\text{cov}(\underline{\tilde{\mathbf{X}}}_u, \underline{\tilde{\mathbf{X}}}_u)$ où $\underline{\tilde{\mathbf{X}}}_u$ est une version centrée et normalisée de $\underline{\mathbf{X}}_u$, et du tenseur de covariance croisée $\text{cov}(\underline{\tilde{\mathbf{X}}}_u, \mathbf{Z}_u)$. Ces tenseurs de covariance et de covariance croisée sont modifiés par :

$$\text{cov}\left(\underline{\tilde{\mathbf{X}}}_u, \underline{\tilde{\mathbf{X}}}_u\right) = \lambda.\text{cov}\left(\underline{\tilde{\mathbf{X}}}_u, \underline{\tilde{\mathbf{X}}}_u\right) + \underline{\tilde{\mathbf{X}}}_u \times_1 \underline{\tilde{\mathbf{X}}}_u \tag{14-1}$$

$$\text{cov}\left(\underline{\tilde{\mathbf{X}}}_u, \mathbf{Z}_u\right) = \lambda.\text{cov}\left(\underline{\tilde{\mathbf{X}}}_u, \mathbf{Z}_u\right) + \underline{\tilde{\mathbf{X}}}_u \times_1 \mathbf{Z}_u \tag{14-2}$$

La méthode REW-NPLS fournit un ensemble de vecteurs de projection $\left\{\mathbf{w}_{u,1},...,\mathbf{w}_{u,n}\right\}_{f=1}^{F}$, de dimensions respectives $I_1,..,I_n$, un ensemble de tenseurs de coefficients de prédiction, $\left\{\underline{\mathbf{B}}_u^f\right\}_{f=1}^{F}$, et un ensemble de vecteurs de biais de prédiction, $\left\{\mathbf{b}_u^f\right\}_{f=1}^{F}$. Le rang $f_{opt}$ conduisant à l'erreur de prédiction minimale est sélectionné, puis le tenseur $\underline{\mathbf{B}}$ ainsi que le vecteur $\mathbf{b}$ sont mis à jour par :

$$\underline{\mathbf{B}} = \underline{\mathbf{B}}_u^{f_{opt}} \tag{15-1}$$

$$\mathbf{b} = \mathbf{b}_u^{f_{opt}} \tag{15-2}$$

Le tenseur $\underline{\mathbf{B}}$ et le vecteur $\mathbf{b}$ sont fournis au module d'estimation de coefficients de mélange, 250.

A partir des éléments $\hat{z}_{k,t}$ du vecteur prédit $\hat{\mathbf{z}}_t$, le module d'estimation d'état peut calculer les probabilités conditionnelles au moyen de la fonction softmax :

$$p\left(z_t = k \mid \underline{\mathbf{X}}_t\right) = \frac{\exp\left(\hat{z}_{k,t}\right)}{\sum_{i=1}^{K} \exp\left(\hat{z}_{i,t}\right)} \tag{16}$$

Cette expression donne la probabilité que l'automate soit dans l'état $k$ à l'instant $t$, connaissant les données d'entrée en cet instant.

Le module d'estimation de coefficients de mélange, 250, obtient ces coefficients à partir de la règle de Bayes :

$$\gamma_k^t = p\left(z_t = k \middle| \underline{\mathbf{X}}_{1:t}\right) = \frac{p\left(z_t = k, \underline{\mathbf{X}}_{1:t}\right)}{p\left(\underline{\mathbf{X}}_{1:t}\right)} = \frac{p\left(z_t = k, \underline{\mathbf{X}}_{1:t}\right)}{\sum_{i=1}^{K} p\left(z_t = i, \underline{\mathbf{X}}_{1:t}\right)} \tag{17}$$

où $p(z_t = i, \underline{\mathbf{X}}_{1:t})$ sont obtenus par l'algorithme *forward*, à savoir :

$$p\left(z_t = i, \underline{\mathbf{X}}_{1:t}\right) = p\left(\underline{\mathbf{X}}_t \middle| z_t = i\right).\sum_{j=1}^{K} a^{ij}\gamma_k^{t-1} \tag{18}$$

En combinant les expressions (17) et (18), les coefficients de mélange peuvent être obtenus par récurrence :

$$\gamma_k^t = \frac{p\left(\underline{\mathbf{X}}_t \middle| z_t = k\right).\sum_{j=1}^{K} a^{kj}\gamma_k^{t-1}}{\sum_{i=1}^{K}\left( p\left(\underline{\mathbf{X}}_t \middle| z_t = i\right).\sum_{j=1}^{K} a^{ij}\gamma_i^{t-1}\right)} \tag{19}$$

Les probabilités conditionnelles d'émission, $p(\underline{\mathbf{X}}_t|z_t = i)$ peuvent être obtenues au moyen de la règle de Bayes à partir des probabilités conditionnelles *a posteriori* $p(z_t = i|\underline{\mathbf{X}}_t)$ et des probabilités *a priori* $p(z_t = i)$ :

$$p\left(\underline{\mathbf{X}}_t \middle| z_t = i\right) = \frac{p\left(z_t = i \middle| \underline{\mathbf{X}}_t\right) p\left(\underline{\mathbf{X}}_t\right)}{p\left(z_t = i\right)} \propto \frac{p\left(z_t = i \middle| \underline{\mathbf{X}}_t\right)}{p\left(z_t = i\right)} \tag{20}$$

le terme $p(\underline{\mathbf{X}}_t)$ étant un coefficient multiplicatif commun à tous les états.

Les probabilités d'occupation des différents états $p(z_t = i)$ à l'instant $t$ sont calculées à partir des probabilités d'occupation initiales $\pi$ et de la matrice de transition **A**.

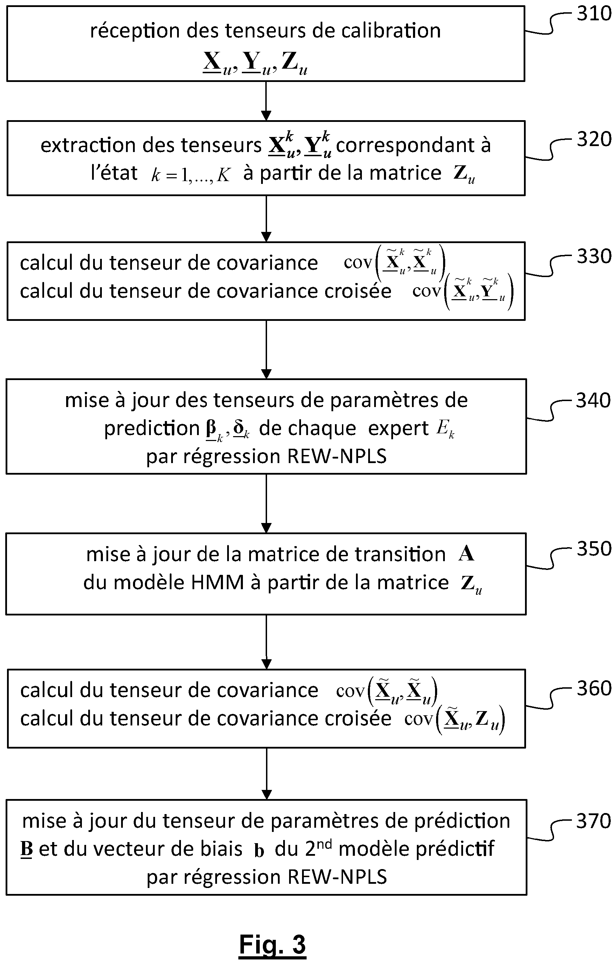

La Fig. 3 représente sous forme d'ordinogramme une méthode de calibration itérative d'une interface neuronale directe à mélange markovien d'experts, selon un mode de réalisation de l'invention.

Ladite méthode de calibration opère lors de phases de calibration prévues en des instants déterminés, par exemple à intervalles de temps réguliers le long de la trajectoire. Chaque phase de calibration u fait intervenir une pluralité de blocs de données d'entrée correspondant à une pluralité $\Delta L$ d'instants successifs de la séquence des instants d'observation. Elle fait également intervenir une pluralité de blocs de sortie donnant les paramètres cinématiques de consigne en ces mêmes instants.

La pluralité de blocs de données d'entrée relatifs à la phase de calibration u est représentée par le tenseur d'entrée $\underline{\mathbf{X}}_u$ et la pluralité de blocs de sortie lors de cette même phase de calibration est représentée par le tenseur de sortie $\underline{\mathbf{Y}}_u$.

On suppose en outre que l'on connaît les états de l'automate HMM lors de la phase de calibration. Les différents états de l'automate HMM pourront être relatifs à différents éléments à commander pour effectuer la trajectoire, par exemples différents membres d'un exosquelette. En outre, pour chacun de ces éléments, différents états pourront être prévus comme par exemple un état actif lorsque le sujet commande cet élément, un état de repos lorsqu'il ne le commande pas et, le cas échéant un état préparatoire précédant immédiatement l'état actif et succédant immédiatement l'état de repos, pendant lesquels les caractéristiques des signaux neuronaux ne sont ni ceux de l'état de repos ni ceux de l'état actif relatif à cet élément. Ainsi lorsque l'interface neuronale directe devra commander $P$ éléments pour effectuer la trajectoire, l'automate HMM comprendra $2^P$ voire $3^P$ états (si des états préparatoires sont envisagés). Les états de l'automate HMM lors de la phase de calibration peuvent être représentés par une matrice binaire $\mathbf{Z}_u$ de taille $K \times \Delta L$, chaque colonne de $\mathbf{Z}_u$ ne comprenant que des 0 sauf un élément égal à 1 indiquant l'état de l'automate à l'instant correspondant.

Le tenseur d'entrée, $\underline{\mathbf{X}}_u$, le tenseur de sortie, $\underline{\mathbf{Y}}_u$, la matrice d'états, $\mathbf{Z}_u$, sont fournis en 310 pour la phase de calibration u .

A l'étape 320, on détermine, à partir de la matrice $\mathbf{Z}_u$, les tenseurs $\underline{\mathbf{X}}_u^k$ et $\underline{\mathbf{Y}}_u^k$ formés respectivement par les blocs de données d'entrée et les blocs de données de sortie, relatifs à l'état $k = 1,..,K$ . Ces tenseurs sont extraits du tenseur d'entrée, $\underline{\mathbf{X}}_u$ , et du tenseur de sortie, $\underline{\mathbf{Y}}_u$ .

A l'étape 330, on calcule, pour chaque expert $E_k$, $k = 1,..,K$ les tenseurs centrés et normalisés $\widetilde{\underline{\mathbf{X}}}_u^k$ et $\widetilde{\underline{\mathbf{Y}}}_u^k$ et on en déduit le tenseur de covariance de $\widetilde{\underline{\mathbf{X}}}_u^k$ , ainsi que le tenseur de covariance croisée de $\widetilde{\underline{\mathbf{X}}}_u^k$ et $\widetilde{\underline{\mathbf{Y}}}_u^k$ . Ces tenseurs sont modifiés en leur ajoutant respectivement le tenseur de covariance de $\widetilde{\underline{\mathbf{X}}}_{u-1}^k$ et de covariance croisée obtenus à la phase de calibration précédente, pondérés par un facteur d'oubli $\lambda^k$. Ces tenseurs ainsi modifiés serviront de tenseur de covariance et de covariance croisée à la phase de calibration suivante.

A l'étape 340, on met à jour les tenseurs de paramètres de prédiction $\underline{\beta}_k$, $\underline{\delta}_k$ de chaque expert $E_k$ au moyen d'une régression REW-NPLS, à partir des tenseurs de covariance et de covariance croisée modifiés à l'étape précédente.

A l'issue de l'étape 340, on dispose d'une version mise à jour de l'ensemble $\theta_e$ des paramètres de prédiction des $K$ experts.

A l'étape 350, on met avantageusement à jour les éléments de la matrice de transition **A** à partir du nombre de transitions entre états successifs observées pendant la phase de calibration $u$ . Le nombre de transitions entre états successifs est obtenu à partir de la matrice $\mathbf{Z}_u$ . On modifie ensuite la matrice **A** en lui ajoutant la matrice **A** obtenue lors de l'itération précédente, pondérée par un facteur d'oubli $\lambda$.

A l'étape 360, on calcule le tenseur centré et normalisé $\tilde{\underline{\mathbf{X}}}_u$, puis le tenseur de covariance de $\tilde{\underline{\mathbf{X}}}_u$ ainsi que le tenseur de covariance croisée de $\tilde{\underline{\mathbf{X}}}_u$ et $\mathbf{Z}_u$. Ces tenseurs sont modifiés en leur ajoutant respectivement le tenseur de covariance de $\tilde{\underline{\mathbf{X}}}_{u-1}$ et de covariance croisée $\tilde{\underline{\mathbf{X}}}_{u-1}$ et $\mathbf{Z}_{u-1}$, obtenus à la phase de calibration précédente, pondérés par le facteur d'oubli $\lambda$. Ces tenseurs ainsi modifiés serviront de tenseur de covariance et de covariance croisée à la phase de calibration suivante.

A l'étape 370, on entraine un modèle prédictif multilinéaire donnant un vecteur d'état de l'automate HMM en fonction du tenseur d'entrée, $\hat{\mathbf{z}}_t = \underline{\mathbf{B}}\underline{\mathbf{X}}_t + \mathbf{b}$, les composantes du vecteur d'état $\hat{\mathbf{z}}_t$ fournissant les probabilités respectives que l'automate occupe les différents états $k=1,..,K$ à l'instant $t$. Plus précisément, on met à jour le tenseur de paramètres de prédiction $\underline{\mathbf{B}}$ et le vecteur de biais b au moyen d'une régression REW-NPLS, à partir des tenseurs de covariance et de covariance croisée modifiés à l'étape précédente.

Les composantes de $\hat{\mathbf{z}}_t$ calculées lors d'une phase opérationnelle permettent ensuite d'obtenir, à chaque instant d'observation $t$, les coefficients de mélange $\gamma_k^t = p\left(z_t = k | \underline{\mathbf{X}}_{1:t}\right)$ des différents experts.

### Revendications

1. Méthode de calibration d'une interface neuronale directe (200) destinée à recevoir une pluralité de signaux électrophysiologiques acquis au moyen d'une pluralité de capteurs (205), pendant une pluralité de fenêtres d'observation associées à des instants d'observation, et à fournir des signaux de commande décrivant une trajectoire à réaliser, lesdits signaux électrophysiologiques subissant un prétraitement pour obtenir un tenseur d'observation ($\underline{\mathbf{X}}_t$) en chaque instant ($t$) d'observation, l'évolution du tenseur d'observation étant modélisée par un modèle Markovien caché, dit modèle HMM, ladite interface utilisant un mélange d'une pluralité $K$ d'experts, chaque expert ($E_k$) étant associé à un état caché ($k$) du modèle HMM et étant défini par un modèle prédictif multilinéaire, les prédictions de chaque expert étant combinées au moyen de coefficients de mélange ( $\gamma_k^t$ , $k = 1,..,K$) pour fournir une estimation ($\hat{\underline{\mathbf{Y}}}_t$) du tenseur de commande représentant lesdits signaux de commande, **caractérisée en ce que** :

   ladite calibration est effectuée lors d'une pluralité de phases de calibration, en des instants prédéterminés de ladite trajectoire, chaque phase de calibration u correspondant à une pluralité ($\Delta L$) d'instants d'observation successifs, et faisant appel (310) à un tenseur $\underline{\mathbf{X}}_u$ représentant le tenseur d'observation en ladite pluralité d'instant successifs, un tenseur $\underline{\mathbf{Y}}_u$ représentant un tenseur de commande de consigne en ces mêmes instants et une matrice $\mathbf{Z}_u$ donnant les états du modèle HMM en ces mêmes instants, et que chaque phase de calibration comprend:

   une étape (a) dans laquelle on extrait (320) des tenseurs $\underline{\mathbf{X}}_u$ et $\underline{\mathbf{Y}}_u$, au moyen de la matrice $\mathbf{Z}_u$, des tenseurs

d'observation $\underline{\mathbf{X}}_u^k$ et de commande $\underline{\mathbf{Y}}_u^k$, $k=1,..,K$, relatifs aux différents états ($k$) du modèle HMM ;

une étape (b) dans laquelle, pour chaque expert $E_k$, on calcule (330) des tenseurs $\underline{\tilde{\mathbf{X}}}_u^k$ et $\underline{\tilde{\mathbf{Y}}}_u^k$ correspondant respectivement aux tenseurs $\underline{\mathbf{X}}_u^k$ et $\underline{\mathbf{Y}}_u^k$, après les avoir centrés et normalisés, puis le tenseur de covariance du tenseur $\underline{\tilde{\mathbf{X}}}_u^k$ ainsi que le tenseur de covariance croisée des tenseurs $\underline{\tilde{\mathbf{X}}}_u^k$ et $\underline{\tilde{\mathbf{Y}}}_u^k$, ces tenseurs de covariance et de covariance croisée étant modifiés en leur ajoutant respectivement les tenseurs de covariance de $\underline{\tilde{\mathbf{X}}}_{u-1}^k$ et de covariance croisée de $\underline{\tilde{\mathbf{X}}}_{u-1}^k$ et $\underline{\tilde{\mathbf{Y}}}_{u-1}^k$ issus de la phase de calibration précédente, pondérés par un facteur d'oubli, $\lambda^k$ ;

une étape (c) dans laquelle on met à jour (340) les modèles prédictifs multilinéaires des experts, $E_k$, $k = 1,..,K$ au moyen d'une régression multivariée des moindres carrés partiels à pondération récursive exponentielle, dite régression REW-NPLS, à partir des tenseurs de covariance et de covariance croisée modifiés à l'étape précédente ;

une étape (d) dans laquelle on calcule (360), le tenseur $\tilde{\mathbf{X}}_u$ correspondant au tenseur $\mathbf{X}_u$, après l'avoir centré et normalisé, puis le tenseur de covariance du tenseur $\tilde{\mathbf{X}}_u$ ainsi que le tenseur de covariance croisée de $\tilde{\mathbf{X}}_u$ et $\mathbf{Z}_u$, ces tenseurs de covariance et de covariance croisée étant modifiés en leur ajoutant respectivement le tenseur de covariance de $\tilde{\mathbf{X}}_{u-1}$ et de covariance croisée de $\tilde{\mathbf{X}}_{u-1}$ et $\mathbf{Z}_{u-1}$, issus de la phase de calibration précédente, pondérés par un facteur d'oubli $\lambda$ ;

une étape (e) dans laquelle on met à jour (370) un second modèle prédictif multilinéaire donnant un vecteur d'état ($\hat{\mathbf{z}}_t$) du modèle HMM en fonction du tenseur d'entrée centré et normalisé, $\tilde{\mathbf{X}}_u$, les composantes du vecteur d'état fournissant les probabilités respectives que le modèle HMM occupe les différents états $k = 1,..,K$ en un instant d'observation, ladite mise à jour étant réalisée au moyen d'une régression REW-NPLS, à partir des tenseurs de covariance et de covariance croisée modifiés à l'étape précédente, les coefficients de mélange ($\gamma_k^t$, $k = 1,..,K$) des différents experts étant ensuite obtenus au moyen du second modèle prédictif multilinéaire.

2. Méthode de calibration d'une interface neuronale directe selon la revendication 1, **caractérisée en ce qu'**à chaque phase de calibration, on détermine le nombre de transitions par couple d'états observées pendant cette phase de calibration et que l'on met à jour la matrice de transition du modèle HMM à partir de ces nombres et de la matrice de transition du modèle HMM calculée lors de la phase de calibration précédente, pondérée par un facteur d'oubli.

3. Méthode de calibration d'une interface neuronale directe selon la revendication 1ou 2, **caractérisée en ce qu'**à l'étape (b), le tenseur modifié de covariance $\mathrm{cov}\left(\underline{\tilde{\mathbf{X}}}_u^k, \underline{\tilde{\mathbf{X}}}_u^k\right)$ du tenseur d'observation relatif à l'état $k$, centré et normalisé, $\underline{\tilde{\mathbf{X}}}_u^k$, est calculé par $\mathrm{cov}\left(\underline{\tilde{\mathbf{X}}}_u^k, \underline{\tilde{\mathbf{X}}}_u^k\right) = \lambda^k \mathrm{cov}\left(\underline{\tilde{\mathbf{X}}}_{u-1}^k, \underline{\tilde{\mathbf{X}}}_{u-1}^k\right) + \underline{\tilde{\mathbf{X}}}_u^k \times_1 \underline{\tilde{\mathbf{X}}}_u^k$ où $\times_1$ est un produit tensoriel relatif au premier mode, et que le tenseur modifié de covariance croisée $\mathrm{cov}\left(\underline{\tilde{\mathbf{X}}}_u^k, \underline{\tilde{\mathbf{Y}}}_u^k\right)$ où $\underline{\tilde{\mathbf{Y}}}_u^k$ est le tenseur de commande relatif à l'état $k$, centré et normalisé, est calculé par $\mathrm{cov}\left(\underline{\tilde{\mathbf{X}}}_u^k, \underline{\tilde{\mathbf{Y}}}_u^k\right) = \lambda^k \mathrm{cov}\left(\underline{\tilde{\mathbf{X}}}_{u-1}^k, \underline{\tilde{\mathbf{Y}}}_{u-1}^k\right) + \underline{\tilde{\mathbf{X}}}_u^k \times_1 \underline{\tilde{\mathbf{Y}}}_u^k$.

4. Méthode de calibration d'une interface neuronale directe selon la revendication 3, **caractérisée en ce qu'**à l'étape (c), pour chaque expert $E_k$, $k = 1,..,K$, le rang optimal, $f_{opt}^k$, de l'espace des variables latentes pour la régression REW-NPLS est déterminé comme celui minimisant l'erreur $err_u^{k,f^k} = \lambda^k err_{u-1}^{k,f^k} + \left\| \mathbf{Y}_u^k - \hat{\mathbf{Y}}_{u-1}^{k,f^k} \right\|$ où $err_u^{k,f^k}$ est l'erreur de prédiction du tenseur de commande relatif à l'état $k$, par l'expert $E_k$ lors de la phase de calibration $u$ pour

un rang $f_k = 1,...,F$ où $F$ est un rang maximal prédéterminé, et $\hat{\underline{\mathbf{Y}}}_{u-1}^{k,f^k}$ est la prédiction de $\underline{\mathbf{Y}}_u^k$ par l'expert $E_k$, obtenue par le modèle prédictif multilinéaire de ce même expert lors de la phase de calibration précédente, pour ce même rang.

5. Méthode de calibration d'une interface neuronale directe selon une des revendications précédentes, **caractérisée en ce qu'**à l'étape (d), le tenseur modifié de covariance $\text{cov}(\underline{\tilde{\mathbf{X}}}_u, \underline{\tilde{\mathbf{X}}}_u)$ du tenseur d'observation, centré et normalisé, $\underline{\tilde{\mathbf{X}}}_u$, est calculé par $\text{cov}(\underline{\tilde{\mathbf{X}}}_u, \underline{\tilde{\mathbf{X}}}_u) = \lambda.\text{cov}(\underline{\tilde{\mathbf{X}}}_u, \underline{\tilde{\mathbf{X}}}_u) + \underline{\tilde{\mathbf{X}}}_u \times_1 \underline{\tilde{\mathbf{X}}}_u$ où $\times_1$ est un produit tensoriel relatif au premier mode, et que le tenseur de covariance croisée $\text{cov}(\underline{\tilde{\mathbf{X}}}_u, \mathbf{Z}_u)$ est modifié par $\text{cov}(\underline{\tilde{\mathbf{X}}}_u, \mathbf{Z}_u) = \lambda.\text{cov}(\underline{\tilde{\mathbf{X}}}_u, \mathbf{Z}_u) + \underline{\tilde{\mathbf{X}}}_u \times_1 \mathbf{Z}_u$ .

6. Méthode de calibration d'une interface neuronale directe selon la revendication 5, **caractérisée en ce qu'**à l'étape (e), le rang optimal, $f_{opt}$, de l'espace des variables latentes pour la régression REW-NPLS est déterminé comme celui minimisant l'erreur $err_u^f = \lambda.err_{u-1}^f + \left\| \underline{\mathbf{Y}}_u - \hat{\underline{\mathbf{Y}}}_{u-1}^f \right\|$ où $err_u^f$ est l'erreur de prédiction du tenseur de commande lors de la phase de calibration $u$ pour un rang $f = 1,...,F$ où $F$ est un rang maximal prédéterminé, et $\hat{\underline{\mathbf{Y}}}_{-u-1}^f$ est la prédiction de $\underline{\mathbf{Y}}_u$ par le mélange d'experts pondérés par des coefficients de mélange obtenus au moyen du second modèle prédictif multilinéaire lors de la phase de calibration précédente, pour ce même rang.

7. Méthode de calibration d'une interface neuronale directe selon l'une des revendications précédentes, **caractérisée en ce que** les signaux électrophysiologiques sont des signaux électrocorticographiques.


## Patentansprüche

1. Verfahren zur Kalibrierung einer direkten neuronalen Schnittstelle (200), die dazu bestimmt ist, eine Vielzahl von elektrophysiologischen Signalen zu empfangen, die mittels einer Vielzahl von Sensoren (205) während einer Vielzahl von Beobachtungszeitpunkten zugeordneten Beobachtungsfenstern erfasst werden, und Steuersignale zu liefern, die eine zu realisierende Trajektorie beschreiben, wobei die elektrophysiologischen Signale einer Vorverarbeitung unterzogen werden, um einen Beobachtungstensor ($\underline{\mathbf{X}_t}$) zu jedem Beobachtungszeitpunkt ($t$) zu erhalten, wobei der Verlauf des Beobachtungstensors durch ein Hidden-Markov-Modell, HMM-Modell genannt, modelliert wird, wobei die Schnittstelle eine Mischung aus einer Vielzahl $K$ von Experten verwendet, wobei jeder Experte ($E_k$) einem verborgenen Zustand (k) des HMM-Modells zugeordnet ist und durch ein multilineares Vorhersagemodell definiert ist, wobei die Vorhersagen jedes Experten mittels Mischungskoeffizienten ($\gamma_k^t$ , $k$ = 1, $K$) kombiniert werden, um eine Schätzung ($\underline{\hat{\mathbf{Y}}_t}$) des die Steuersignale repräsentierenden Steuertensors zu liefern, **dadurch gekennzeichnet, dass**:

   die Kalibrierung während einer Vielzahl von Kalibrierungsphasen an vorbestimmten Zeitpunkten der Trajektorie durchgeführt wird, wobei jede Kalibrierungsphase $u$ einer Vielzahl ($\Delta L$) von aufeinanderfolgenden Beobachtungszeitpunkten entspricht und auf einen Tensor $\underline{\mathbf{X}_u}$, der den Beobachtungstensor an der Vielzahl von aufeinanderfolgenden Zeitpunkten darstellt, einen Tensor $\underline{\mathbf{Y}_u}$, der einen Soll-Steuertensor an denselben Zeitpunkten darstellt, und eine Matrix $\mathbf{Z}_u$, die die Zustände des HMM-Modells an denselben Zeitpunkten angibt, zurückgreift (310), und dass jede Kalibrierungsphase umfasst:

   einen Schritt (a), bei dem aus den Tensoren $\underline{\mathbf{X}_u}$ und $\underline{\mathbf{Y}_u}$ mittels der Matrix $\mathbf{Z}_u$ die Beobachtungstensoren $\underline{\mathbf{X}}_u^k$ und die Steuertensoren $\underline{\mathbf{Y}}_u^k$, $k$ = 1,...,$K$, die sich auf die verschiedenen Zustände (k) des HMM-Modells beziehen, extrahiert werden (320);

   einen Schritt (b), bei dem für jeden Experten $E_k$ die Tensoren $\underline{\tilde{\mathbf{X}}}_u^k$ und $\underline{\tilde{\mathbf{Y}}}_u^k$ berechnet werden (330), die jeweils den Tensoren $\underline{\mathbf{X}}_u^k$ bzw. $\underline{\mathbf{Y}}_u^k$ entsprechen, nachdem sie zentriert und normalisiert wurden, dann den Kovarianz-Tensor des Tensors $\underline{\tilde{\mathbf{X}}}_u^k$ sowie den Kreuzkovarianz-Tensoren der Tensoren $\underline{\tilde{\mathbf{X}}}_u^k$ und $\underline{\tilde{\mathbf{Y}}}_u^k$, wobei diese Kovarianz- und Kreuzkovarianz-Tensoren modifiziert werden, indem man ihnen jeweils die Kovarianz-Tensoren $\underline{\tilde{\mathbf{X}}}_{u-1}^k$ bzw. Kreuzkovarianz-Tensoren $\underline{\tilde{\mathbf{X}}}_{u-1}^k$ und $\underline{\tilde{\mathbf{Y}}}_{u-1}^k$ aus der vorherigen Kalibrierungsphase, gewichtet

mit einem Vergessensfaktor $\lambda^k$, hinzufügt;

einen Schritt (c), bei dem man die multilinearen Vorhersagemodelle der Experten, $E_k$, $k = 1,..., K$ mittels einer multivariaten Regression der kleinsten partiellen Quadrate mit exponentiell rekursiver Gewichtung, REW-NPLS-Regression genannt, ausgehend von den im vorherigen Schritt modifizierten Kovarianz- und Kreuzkovarianz-Tensoren aktualisiert (340);

einen Schritt (d), bei dem der Tensor $\tilde{\underline{X}}_u$, der dem Tensor $\underline{X_u}$ entspricht, nachdem er zentriert und normalisiert wurde, dann der Kovarianztensor des Tensors $\tilde{\underline{X}}_u$ sowie der Kreuzkovarianz-Tensor von $\tilde{\underline{X}}_u$ und $Z_u$, berechnet werden (360), wobei diese Kovarianz- und Kreuzkovarianz-Tensoren modifiziert werden, indem ihnen jeweils der Kovarianz-Tensor von $\tilde{\underline{X}}_{u-1}$ und der Kreuzkovarianz-Tensor von $\tilde{\underline{X}}_{u-1}$ und $Z_{u-1}$ aus der vorherigen Kalibrierungsphase, gewichtet mit einem Vergessensfaktor $\lambda$, hinzugefügt werden;

einen Schritt (e), bei dem ein zweites multilineares Vorhersagemodell aktualisiert wird (370), das einen Zustandsvektor ($\hat{z}_t$) des HMM-Modells als Funktion des zentrierten und normalisierten Eingangstensors $\underline{X}_u$ ergibt, wobei die Komponenten des Zustandsvektors die jeweiligen Wahrscheinlichkeiten liefern, dass das HMM-Modell die verschiedenen Zustände $k = 1, ..., K$ zu einem Beobachtungszeitpunkt einnimmt, wobei die Aktualisierung mittels einer REW-NPLS-Regression ausgehend von den im vorherigen Schritt modifizierten Kovarianz- und Kreuzkovarianz-Tensoren erfolgt, wobei die Mischungskoeffizienten ($\gamma_k^t$, $k = 1,...,K$) der verschiedenen Experten dann mit Hilfe des zweiten multilinearen Vorhersagemodells erhalten werden.

2. Verfahren zur Kalibrierung einer direkten neuronalen Schnittstelle nach Anspruch 1, **dadurch gekennzeichnet, dass** in jeder Kalibrierungsphase die Anzahl der während dieser Kalibrierungsphase beobachteten Übergänge pro Zustandspaar bestimmt wird und dass die Übergangsmatrix des HMM-Modells anhand dieser Zahlen und der in der vorherigen Kalibrierungsphase berechneten, mit einem Vergessensfaktor gewichteten Übergangsmatrix des HMM-Modells aktualisiert wird.

3. Verfahren zur Kalibrierung einer direkten neuronalen Schnittstelle nach Anspruch 1 oder 2, **dadurch gekennzeichnet, dass** in Schritt (b) der modifizierte Kovarianz-Tensor cov ($\tilde{\underline{X}}_u^k$, $\tilde{\underline{X}}_u^k$) des auf den Zustand $k$ bezogenen, zentrierten und normalisierten Beobachtungstensors $\tilde{\underline{X}}_u^k$ berechnet wird durch

$$\text{cov}(\tilde{\underline{X}}_u^k, \tilde{\underline{X}}_u^k) = \lambda^k \text{ cov}(\tilde{\underline{X}}_{u-1}^k, \tilde{\underline{X}}_{u-1}^k) + \tilde{\underline{X}}_u^k \times_1 \tilde{\underline{X}}_u^k$$

, worin $\times_1$ ein Tensorprodukt bezüglich des ersten Modus ist, und dass der modifizierte Kreuzkovarianz-Tensor cov ($\tilde{\underline{X}}_u^k$, $\tilde{\underline{Y}}_u^k$), worin $\tilde{\underline{Y}}_u^k$ der auf den Zustand $k$ bezogene, zentrierte und normalisierte Steuertensor ist, durch

$$\text{cov}(\tilde{\underline{X}}_u^k, \tilde{\underline{Y}}_u^k) = \lambda^k \text{ cov}(\tilde{\underline{X}}_{u-1}^k, \tilde{\underline{Y}}_{u-1}^k) + \tilde{\underline{X}}_u^k \times_1 \tilde{\underline{Y}}_u^k$$

berechnet wird.

4. Verfahren zur Kalibrierung einer direkten neuronalen Schnittstelle nach Anspruch 3, **dadurch gekennzeichnet, dass** in Schritt (c) für jeden Experten $E_k$, $k = 1, ..., K$ der optimale Rang $f_{opt}^k$ des Raums der latenten Variablen für die REW-NPLS-Regression als derjenige bestimmt wird, der den Fehler

$$err_u^{k,f^k} = \lambda^k \ err_{u-1}^{k,f^k} + \left\| \underline{Y}_u^k - \hat{\underline{Y}}_{u-1}^{k,f^k} \right\|$$

minimiert, worin $err_u^{k,f^k}$ der Fehler der Vorhersage des auf den Zustand $k$ bezogenen Steuertensors durch den Experten $E_k$ in der Kalibrierungsphase u für einen Rang $f_k = 1, ..., F$ ist, worin $F$ ein vorbestimmter maximaler Rang ist, und $\hat{\underline{Y}}_{u-1}^{k,f^k}$ die Vorhersage von $\underline{Y}_u^k$ durch den Experten $E_k$ ist, die durch das multilineare Vorhersagemodell desselben Experten in der vorherigen Kalibrierungsphase für denselben Rang erhalten wurde.

5. Verfahren zur Kalibrierung einer direkten neuronalen Schnittstelle nach einem der vorhergehenden Ansprüche, **dadurch gekennzeichnet, dass** in Schritt (d) der modifizierte Kovarianz-Tensor cov ($\tilde{\underline{X}}_u$, $\tilde{\underline{X}}_u$) des zentrierten und normalisierten Beobachtungstensors $\tilde{\underline{X}}_u$ berechnet wird durch cov ($\tilde{\underline{X}}_u$, $\tilde{\underline{X}}_u$) = $\lambda.\text{cov}$ ($\tilde{\underline{X}}_u$, $\tilde{\underline{X}}_u$) + $\tilde{\underline{X}}_u \times_1 \tilde{\underline{X}}_u$, worin $\times_1$ ein Tensorprodukt bezüglich des ersten Modus ist, und dass der Kreuzkovarianz-Tensor cov ($\tilde{\underline{X}}_u$, $Z_u$) durch cov ($\tilde{\underline{X}}_u$, $Z_u$) = $\lambda.\text{cov}$ ($\tilde{\underline{X}}_u$, $Z_u$) + $\underline{X}_u \times_1 Z_u$ modifiziert wird.

6. Verfahren zur Kalibrierung einer direkten neuronalen Schnittstelle nach Anspruch 5, **dadurch gekennzeichnet, dass** in Schritt (e) der optimale Rang $f_{opt}$ des Raums der latenten Variablen für die REW-NPLS-Regression als derjenige bestimmt wird, der den Fehler $err_u^f = \lambda.err_{u-1}^f + \left\| \underline{Y}_u - \underline{\widehat{Y}}_{u-1}^f \right\|$ minimiert, worin $err_u^f$ der Fehler der Vorhersage des Steuertensors in der Kalibrierungsphase u für einen Rang $f$ = 1, ..., $F$ ist, worin $F$ ein vorbestimmter maximaler Rang ist und $\underline{\widehat{Y}}_{u-1}^f$ die Vorhersage von $\underline{Y}_u$ durch die mit Mischungskoeffizienten gewichtete Expertenmischung ist, die mit Hilfe des zweiten multilinearen Vorhersagemodells in der vorhergehenden Kalibrierungsphase für denselben Rang erhalten wurden.

7. Verfahren zur Kalibrierung einer direkten neuronalen Schnittstelle nach einem der vorhergehenden Ansprüche, **dadurch gekennzeichnet, dass** die elektrophysiologischen Signale elektrokortikographische Signale sind.

## Claims

1. A method for calibrating a direct neural interface (200) intended to receive a plurality of electrophysiological signals acquired by means of a plurality of sensors (205), during a plurality of observation windows associated with observation times, and to provide control signals describing a trajectory to be carried out, said electrophysiological signals undergoing a pre-processing to obtain an observation tensor ($\underline{X}_t$) at each observation time ($t$), the evolution of the observation tensor being modelled by a hidden Markovian model, called HMM model, said interface using a mixture of a plurality $K$ of experts, each expert ($E_k$) being associated with a hidden state ($k$) of the HMM model and being defined by a multilinear predictive model, the predictions of each expert being combined by means of mixing coefficients ($\gamma_k^t$, $k$ = 1,.., $K$) to provide an estimate ($\hat{\underline{Y}}_t$) of the control tensor representing said control signals, **characterised in that**:
said calibration is performed during a plurality of calibration phases, at predetermined times of said trajectory, each calibration phase u corresponding to a plurality ($\Delta L$) of successive observation times, and using (310) a tensor $\underline{X}_u$, representing the observation tensor at said plurality of successive times, a tensor $\underline{Y}_u$, representing a setpoint control tensor at these same times and a matrix $Z_u$, giving the states of the HMM model at these same times, and that each calibration phase comprises:

a step (a) in which tensors $\underline{X}_u$ and $\underline{Y}_u$ are extracted (320), by means of the matrix $Z_u$, observation $\underline{X}_u^k$ and control $\underline{Y}_u^k$ tensors, $k$ = 1,.., $K$, relating to the different states (k) of the HMM model;
a step (b) in which, for each expert $E_k$, tensors $\underline{\widetilde{X}}_u^k$ and $\underline{\widetilde{Y}}_u^k$, corresponding respectively to the tensors $\underline{X}_u^k$ and $\underline{Y}_u^k$, are calculated (330), after having centred and normalised them, then the covariance tensor of the tensor $\underline{\widetilde{X}}_u^k$ as well as the cross-covariance tensor of the tensors $\underline{\widetilde{X}}_u^k$ and $\underline{\widetilde{Y}}_u^k$, these covariance and cross-covariance tensors being modified by adding thereto respectively the covariance tensor of $\underline{\widetilde{X}}_{u-1}^k$ and cross-covariance tensor of $\underline{\widetilde{X}}_{u-1}^k$ and $\underline{\widetilde{Y}}_{u-1}^k$ from the previous calibration phase, weighted by a forgetting factor, $\lambda^k$;
a step (c) in which the multilinear predictive models of the experts, $E^k$, $k$ = 1,.., $K$ are updated (340) by means of a multivariate regression of the partial least squares with exponential recursive weighting, called REW-NPLS regression, from the covariance and cross-covariance tensors modified in the previous step;
a step (d) in which, the tensor $\underline{\widetilde{X}}_u$ corresponding to the tensor $\underline{X}_u$, is calculated (360), after having centred and normalised it, then the covariance tensor of the tensor $\underline{\widetilde{X}}_u$, as well as the cross-covariance tensor of $\underline{\widetilde{X}}_u$ and $Z_u$, these covariance and cross-covariance tensors being modified by adding thereto respectively the covariance tensor of $\underline{\widetilde{X}}_{u-1}$ and the cross-covariance tensor of $\underline{\widetilde{X}}_{u-1}$ and $Z_{u-1}$, from the previous calibration phase, weighted by a forgetting factor $\lambda$;

a step (e) in which a second multilinear predictive model is updated (370) giving a state vector ($\hat{\underline{z}}_t$) of the HMM model depending on the centred and normalised input tensor, $\underline{\widetilde{X}}_u$, the components of the state vector providing the respective probabilities that the HMM model occupies the different states $k$ = 1,..,$K$ in an observation time, said update being carried out by means of a REW-NPLS regression, from the covariance and cross-covariance tensors modified in the previous step, the mixing coefficients ( $\gamma_k^t$ , $k$=1,..,$K$) of the different experts then being obtained by means of the second multilinear predictive model.

2. The method for calibrating a direct neural interface according to claim 1, **characterised in that** in each calibration phase, the number of transitions per pair of states, which are observed during this calibration phase, is determined and the transition matrix of the HMM model is updated from these numbers and the transition matrix of the HMM model calculated during the previous calibration phase, weighted by a forgetting factor.

3. The method for calibrating a direct neural interface according to claim 1 or 2, **characterised in that** in step (b), the modified covariance tensor $\operatorname{cov}(\underline{\widetilde{\mathbf{X}}}_u^k, \underline{\widetilde{\mathbf{X}}}_u^k)$ of the observation tensor relating to the state $k$, centred and normalised, $\underline{\widetilde{\mathbf{X}}}_u^k$, is calculated by $\operatorname{cov}(\underline{\widetilde{\mathbf{X}}}_u^k, \underline{\widetilde{\mathbf{X}}}_u^k) = \lambda^k \operatorname{cov}(\underline{\widetilde{\mathbf{X}}}_{u-1}^k, \underline{\widetilde{\mathbf{X}}}_{u-1}^k) + \underline{\widetilde{\mathbf{X}}}_u^k \mathbf{x}_1 \underline{\widetilde{\mathbf{X}}}_u^k$ , where $\mathbf{x}_1$ is a tensor product relating to the first mode, and that the modified cross-covariance tensor $\operatorname{cov}( \underline{\widetilde{\mathbf{X}}}_u^k, \underline{\widetilde{\mathbf{Y}}}_u^k)$ where $\underline{\widetilde{\mathbf{Y}}}_u^k$ is the control tensor relating to the state $k$, centred and normalised, is calculated by

$$\operatorname{cov}( \underline{\widetilde{\mathbf{X}}}_u^k, \underline{\widetilde{\mathbf{Y}}}_u^k) = \lambda^k \operatorname{cov}(\underline{\widetilde{\mathbf{X}}}_{u-1}^k, \underline{\widetilde{\mathbf{Y}}}_{u-1}^k) + \underline{\widetilde{\mathbf{X}}}_u^k \mathbf{x}_1 \underline{\widetilde{\mathbf{Y}}}_u^k ,$$

4. The method for calibrating a direct neural interface according to claim 3, **characterised in that** in step (c), for each expert, $E_k$, $k = 1,.., K$, the optimal rank, $f_{opt}^k$, of the latent variable space for the REW-NPLS regression is determined as that minimising the error $err_u^{k,f^k}$ , $\lambda^k err_{u-1}^{k,f^k} + \left\| \underline{\mathbf{Y}}_u^k - \underline{\widehat{\mathbf{Y}}}_{u-1}^{k,f^k} \right\|$ where $err_{u-1}^{k,f^k}$ is the prediction error of the control tensor relating to the state $k$, by the expert $E_k$ during the calibration phase u for a rank $f_k$ =1,..,$F$, where $F$ is a predetermined maximum rank, and $\underline{\widehat{\mathbf{Y}}}_{u-1}^{k,f^k}$ is the prediction of $\underline{\mathbf{Y}}_u^k$ by expert $E_k$, obtained by the multilinear predictive model of this same expert during the previous calibration phase, for this same rank.

5. The method for calibrating a direct neural interface according to one of the preceding claims, **characterised in that** in step (d), the modified covariance tensor $\operatorname{cov}(\underline{\widetilde{\mathbf{X}}}_u, \underline{\widetilde{\mathbf{X}}}_u)$ of the observation tensor, centred and normalised, $\underline{\widetilde{\mathbf{X}}}_u$, is calculated by $\operatorname{cov}(\underline{\widetilde{\mathbf{X}}}_u, \underline{\widetilde{\mathbf{X}}}_u) = \lambda. \operatorname{cov}(\underline{\widetilde{\mathbf{X}}}_u, \underline{\widetilde{\mathbf{X}}}_u) + \underline{\widetilde{\mathbf{X}}}_u \mathbf{x}_1 \underline{\widetilde{\mathbf{X}}}_u$ where $\mathbf{x}_1$, is a tensor product relating to the first mode, and that the cross-covariance tensor $\operatorname{cov}(\underline{\widetilde{\mathbf{X}}}_u, \mathbf{Z}_u)$ is modified by $\operatorname{cov}(\underline{\widetilde{\mathbf{X}}}_u, \mathbf{Z}_u) = \lambda. \operatorname{cov}(\underline{\widetilde{\mathbf{X}}}_u, \mathbf{Z}_u) + \underline{\widetilde{\mathbf{X}}}_u \mathbf{x}_1 \mathbf{Z}_u$.

6. The method for calibrating a direct neural interface according to claim 5, **characterised in that** in step (e), the optimal rank, $f_{opt}$, of the latent variable space for the REW-NPLS regression is determined as that minimising the error $err_u^f$ , $\lambda. err_{u-1}^f + \left\| \underline{\mathbf{Y}}_u - \underline{\widehat{\mathbf{Y}}}_{u-1}^f \right\|$ where $err_u^f$ is the prediction error of the control tensor during the calibration phase $u$ for a rank $f = 1,.., F$, where $F$ is a predetermined maximum rank, and $\underline{\widehat{\mathbf{Y}}}_{-u-1}^f$ is the prediction of $\underline{\mathbf{Y}}_u$ by the mixture of experts weighted by mixing coefficients which are obtained by means of the second multilinear predictive model during the previous calibration phase, for this same rank.

7. The method for calibrating a direct neural interface according to one of the preceding claims, **characterised in that** the electrophysiological signals are electrocorticographic signals.

**Fig. 1**

**Fig. 2**

```
┌─────────────────────────────────────────────────────┐
│         réception des tenseurs de calibration         │── 310
│                                                       │
│              $\underline{\mathbf{X}}_u, \underline{\mathbf{Y}}_u, \mathbf{Z}_u$                          │
└─────────────────────────────────────────────────────┘
                          │
                          ▼
┌─────────────────────────────────────────────────────┐
│   extraction des tenseurs $\underline{\mathbf{X}}_u^k, \underline{\mathbf{Y}}_u^k$ correspondant à     │── 320
│     l'état  $k = 1,...,K$  à partir de la matrice  $\mathbf{Z}_u$   │
└─────────────────────────────────────────────────────┘
                          │
                          ▼
┌─────────────────────────────────────────────────────┐
│ calcul du tenseur de covariance  $\mathrm{cov}\left(\underline{\widetilde{\mathbf{X}}}_u^k, \underline{\widetilde{\mathbf{X}}}_u^k\right)$       │── 330
│ calcul du tenseur de covariance croisée  $\mathrm{cov}\left(\underline{\widetilde{\mathbf{X}}}_u^k, \underline{\widetilde{\mathbf{Y}}}_u^k\right)$ │
└─────────────────────────────────────────────────────┘
                          │
                          ▼
┌─────────────────────────────────────────────────────┐
│     mise à jour des tenseurs de paramètres de         │── 340
│     prediction $\underline{\boldsymbol{\beta}}_k, \underline{\boldsymbol{\delta}}_k$ de chaque  expert $E_k$        │
│              par régression REW-NPLS                   │
└─────────────────────────────────────────────────────┘
                          │
                          ▼
┌─────────────────────────────────────────────────────┐
│     mise à jour de la matrice de transition  $\mathbf{A}$       │── 350
│     du modèle HMM à partir de la matrice  $\mathbf{Z}_u$        │
└─────────────────────────────────────────────────────┘
                          │
                          ▼
┌─────────────────────────────────────────────────────┐
│ calcul du tenseur de covariance  $\mathrm{cov}\left(\underline{\widetilde{\mathbf{X}}}_u, \underline{\widetilde{\mathbf{X}}}_u\right)$       │── 360
│ calcul du tenseur de covariance croisée  $\mathrm{cov}\left(\underline{\widetilde{\mathbf{X}}}_u, \mathbf{Z}_u\right)$ │
└─────────────────────────────────────────────────────┘
                          │
                          ▼
┌─────────────────────────────────────────────────────┐
│ mise à jour du tenseur de paramètres de prédiction    │── 370
│ $\underline{\mathbf{B}}$ et du vecteur de biais  $\mathbf{b}$  du 2nd modèle prédictif  │
│              par régression REW-NPLS                   │
└─────────────────────────────────────────────────────┘
```

**Fig. 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3053495 A
- FR 3046471 A
- FR 3061318 A

**Littérature non-brevet citée dans la description**

- **M. VELLISTE et al.** Motor cortical correlates of arm resting in the context of a reaching task and implications for prosthetic control. *The Journal of Neuroscience,* 23 Avril 2014, vol. 34 (17), 6011-6022
- **L. SRINIVASAM et al.** General-purpose filter design for neural prosthetic devices. *J. Neurophysiol,* 2007, vol. 98, 2456-2475
- **A. ELISEYEV et al.** Recursive exponentially weighted N-way Partial Least Squares regression with recursive validation of hyper-parameters in Brain Computer Interface applications. *Nature, Scientific Reports,* 24 Novembre 2017, vol. 7 (16281